# EUROPEAN PATENT SPECIFICATION

(11) **EP 2 101 732 B1**
(45) Date of publication and mention of the grant of the patent: **29.05.2019**
(21) Application number: 07817403.4
(22) Date of filing: 27.11.2007
(51) Int. Cl.: A61P 35/00, A61K 31/555, A61N 5/06, A61K 9/00, A61K 47/36, A61K 9/127, A61K 31/409

(54) **LIPOSOMAL GEL PHTHALOCYANINE PREPARATION FOR PHOTODYNAMIC THERAPY OF TUMORS AND ITS MANUFACTURING**
LIPOSOMALE GEL-PHTHALOCYANIN-ZUBEREITUNG FÜR DIE PHOTODYNAMISCHE THERAPIE VON TUMOREN UND IHRE HERSTELLUNG
PRÉPARATION EN GEL LIPOSOMIQUE À BASE DE PHTALOCYANINE POUR THÉRAPIE PHOTODYNAMIQUE DE TUMEURS ET FABRICATION DE CETTE PRÉPARATION

(30) Priority: 28.11.2006 CZ 20060743
(43) Date of publication of application: 23.09.2009
(73) Proprietor: Institute of Physiology of AS CR, v.v.i., 142 20 Praha 4 (CZ); RCD, s.r.o., 252 29 Dobrichovice (CZ)
(72) Inventor: JEZEK, Petr, 196 00 Praha 9 - Cakovice (CZ); NEKVASIL, Milos, 132 00 Praha 3 (CZ); POUCKOVA, Pavla, 252 29 Dobrichovice (CZ)
(74) Representative: Beetz & Partner mbB
(86) International application number: PCT/CZ2007/000107
(87) International publication number: WO 2008/074267

(56) References cited:
- US-A- 5 466 468
- US-A- 5 616 602
- US-A- 5 723 148
- RIBEIRO ET AL: "[1,2,3,4-Tetrakis(alpha/beta-d-galactopyr anos-6-yl)phthalocyaninato]z inc(II): a water-soluble phthalocyanine" TETRAHEDRON LETTERS, ELSEVIER, AMSTERDAM, NL, vol. 47, no. 52, 23 November 2006 (2006-11-23), pages 9177-9180, XP005776913 ISSN: 0040-4039
- POSTIGO ET AL: "Photosensitization of skin fibroblasts and HeLa cells by three chlorin derivatives: Role of chemical structure and delivery vehicle" May 2006 (2006-05), BIOCHIMICA ET BIOPHYSICA ACTA. BIOMEMBRANES, AMSTERDAM, NL, PAGE(S) 583-596 , XP005518282 ISSN: 0005-2736 the whole document
- SIBATA M N ET AL: "Photophysicals and photochemicals studies of zinc(II) phthalocyanine in long time circulation micelles for Photodynamic Therapy use" October 2004 (2004-10), EUROPEAN JOURNAL OF PHARMACEUTICAL SCIENCES, ELSEVIER, AMSTERDAM, NL, PAGE(S) 131-138 , XP004580036 ISSN: 0928-0987 the whole document

## Description

### Technical fields

The technical solution concerns an application gel with liposomes for photodynamic therapy of tumor diseases, which contains a hydrophobic form of hydroxyl-aluminum phthalocyanine (or aluminum substituted by Si, Zn, and other metals or without a metal core) (referred further to as FCH) modified for subsequent treatment by a microfluidizer. The resulting gel containing liposomes with the incorporated curing drug is applied as a therapy on surface tumors in dermatology or on other tumors accessible to light or lightguides, and after several minutes is illuminated by light of a desired wavelength. The suggested system allows instant penetration of the curing drug into the tumor and nearly instant illumination (in minute intervals from the application) with following disintegration effect on the tumor. This highly efficient disintegration result of the therapy is determined by the suggested system.

### Background art

Photodynamic therapy, used in a cure of surface tumors, especially in dermatology, lies in a procedure, that the curing drug is incorporated in a gel, which is applied onto a tumor and after a sufficient time illuminated by the light of the desired wavelength.

For photodynamic therapy of tumor diseases were developed following hydrophobic preparations clinically tested (some only pre-clinically) for intervals between application and illumination (T_{DL}) in a range from one hour and longer (up to several days, see data in parentheses): benzyl ester of delta aminolevulic acid, Benzvix (T_{DL} 4 to 6 hours) registered in EU for therapy of gastrointestinal tumors, U.S. patent 6492420, (comp. Photocure, Oslo, Norway); Temoporfin or Foscan (methyl-tetrahydroxyphenyl chlorine, (TDL 96 hours), WO 0166550, Biolitec Pharma, Scotland, United Kingdom) approved in EU for palliative head and neck tumors, prostatic tumors and pancreatic tumors; Benzoporphyrine derivative, alias Verteporfin (BPD-MA, Visudyne, Novartis, UK), which is in phase III of clinical testing for skin amelanotic melanomas; and silicone phthalocyanine also in phase III of clinical testing for curing of skin tumors including Bowen diseases and actinic keratosis, so far with shortest T_{DL} 1 hour.

The suggested solution of a system of hydrophobic phthalocyanine incorporated in liposomes by microfluidization and applied in a translucent gel exhibited T_{DL} of 5 to 15 minutes in preclinical testing.

### Disclosure of the invention

The invention is achieved as set out in the appended claims.

Liposomal gel hydrophobic phthalocyanine (FCH) preparation for photodynamic therapy of tumor diseases is composed by a system of lecithin liposomes or liposomes on the basis of other lipids, with incorporated curing drug, chosen from a group including hydrophobic hydroxyaluminum phthalocyanine, hydrophobic aluminum phthalocyanine, hydrophobic zinc phthalocyanine, hydrophobic silicone phthalocyanine or organic silicone phthalocyanine, or hydrophobic phthalocyanine without the core metal, mixed in ratios of 10:1 to 1 :10 with a translucent gel, advantageously on the basis of carboxymethylcellulose. The incorporated curing drug can be coated by glucose or other saccharides, by polyethyleneglycole, by lecithin or other lipids, or by sodium chloride or other salts sufficient in pharmacology. The Liposomal gel hydrophobic phthalocyanine (FCH) preparation is manufactured so that the lecithin or other pharmaceutical pure lipid in a concentration of 1 to 40 mg per milliliter of fluid, advantageously fluid as a sterile isotonic solution, is microfluidized on a microfluidizer in a proper chamber until the final particle size smaller than 1000 nanometers, at the temperature higher than 0 °C and a pressure of 500 - 2000 bar; afterwards while constantly stirring the curing drug is added or the treated curing drug according to the points 2 to 5 at ratios between 5:1 to 0.1:1 in relation to the lecithin (lipid); the resulting suspension is again microfluidized on a microfluidizer in proper smaller chamber until the final particle size smaller than 500 nm, a pressure of 1000 - 2000 Bar, and at the temperature higher than 0 0C; the resulting suspension is afterwards mixed with a translucent pharmaceutical gel in ratios between 10:1 to 1:10; alternatively lecithin or other lipid of a pharmaceutical purity in concentration of 1 to 40 mg per ml of fluid, advantageously fluid as a sterile isotonic solution, is microfluidized on a microfluidizer in a proper chamber until the final particle size smaller than 1000 nm, a pressure minimum of 1000 - 2000 Bar, at the temperature higher than 0 °C; afterwards the curing drug or the treated curing drug according to the points 2 to 5 of the claims is solitary microfluidized on a microfluidizer in a desired chamber in amounts corresponding to 5:1 to 0.1 :1 ratios related to the lecithin (lipid) in an equal volume of fluid, advantageously of isotonic solution to the final particle size smaller than 1000 nm and a pressure minimum of 1000 - 2000 Bar, afterwards both microfluidized components are mixed together and again microfluidized on a microfluidizer in the proper smaller chamber with a pressure minimum of 1000 - 2000 Bar, at the temperature higher than 0 °C until the final particle size maximum of 500 nm; the resulting suspension is microfluidized on a microfluidizer in the proper smaller chamber with a pressure minimum of 1000 -2000 Bar, at the temperature higher than 0 0C until the final particle size smaller than 500 nm; the resulting fluid is then mixed with a translucent pharmaceutical gel in ratios of 10:1 to 1:10; or, alternatively, lecithin or other lipid in a pharmaceutical purity at the concentration of 1 to 40 mg per milliliter of fluid, advantageously fluid as a sterile isotonic solution, is treated by extrusion through the filters of sizes 10 to 500 nm together with a curing drug or the treated curing drug according to the points 2 to 5 of the claims in ratios of 5:1 to 0,1:1 related to the lecithin (lipid); the resulting suspension is further microfluidized on a microfluidizer in the proper smaller chamber to the final particle size maximum of 500 nm with a pressure of 1000 -2000 Bar, at the temperature higher than 0 0C until the final particle size smaller than 500 nm with a pressure of 1000 - 2000 Bar, at the temperature higher than 0 °C; the resulting suspension is then mixed with a translucent pharmaceutical gel in ratios of 10:1 to 1:10.

Liposomal gel hydrophobic phthalocyanine preparation for photodynamic therapy of tumors and other diseases; The approach in therapeutic use is that the preparation is applied onto the tumor surface or the pathological part of the body and is let to act for the time period of one minute up to 24 hours, and afterwards, the location is irradiated by the light of the wavelength between 500 to 800 nm and intensity of at least 1 J/cm2. The resulting gel containing liposomes with a curing drug is during therapy applied onto surface tumors in dermatology or other tumors accessible for light-delivering endoscopes and ideally after several minutes is irradiated by light of the desired wavelength. The suggested system enables instant penetration of the curing drug into the tumor and nearly immediate irradiation (in a minute time intervals from the application) with disintegration curing effect on the tumor. Such a high disintegration effect of the suggested therapy is determined by the suggested composition of the gel.

### Examples of technology

### Examples of microfluidization procedure:

### Example #1

On a microfluidizer, i.e. an instrument from Microfluidics, Inc., USA, of a laboratory or industrial type, there is at first microfluidized a powder lecithin of a pharmacological purity at concentration between 10 to 30 mg per ml of sterile isotonic solution. Microfluidization is conducted e.g. in the Z-chamber of 100 micrometer in diameter by several cycles so that the whole volume of the fluid is by several times cycled across the microfluidization chamber, at a pressure of at least 1000 Bar. Afterwards, while constantly stirring the curing powder or the treated curing powder FCH is added according to the points 2 to 5 at ratios between 2:1 in relation to the lecithin. Subsequently, the suspension is again microfluidized in the Z-chamber of 100 micrometer in diameter and at least by 100 cycles passages of the whole volume of fluid at the pressure of more than 1500 Bar and slush cooling. The fourth step is microfluidization in a Z-chamber of 50 micrometer in diameter by at least 100 cycle passages of the fluid at the pressure of more than 1500 Bar and slush cooling.

### Example #2

On a microfluidizer, i.e. an instrument from Microfluidics, Inc., USA, of a laboratory or industrial type, there is at first microfluidized the powder lecithin of a pharmacological purity at concentration between 10 to 30 mg per ml of sterile isotonic solution. Microfluidization is conducted in the Z-chamber of 100 micrometer in diameter by several cycles so that the whole volume of fluid is by several times cycled across the microfluidization chamber, at a pressure of more than 1000 Bar. Afterwards, in parallel the curing powder or the treated curing powder FCH is microfluidized (usually in ratio to 2:1 related to the lecithin in an equal volume of isotonic solution), on a microfluidizer in the Y-chamber of 100 or 75 micrometer in diameter by at least 100 cycle passages of the fluid at a pressure of more than 1500 Bar and slush cooling. Afterwards both microfluidized components are mixed together in the Z-chamber of 100 micrometer in diameter by at least 100 cycle passages of the fluid at a pressure of more than 1500 Bar and slush cooling. The last step is again microfluidization on a microfluidizer in the Z-chamber of 50 micrometer in diameter by at least 100 cycle passages of the fluid at a pressure of more than 1500 Bar and slush cooling.

### Example #3

On a microfluidizer, i.e. an instrument from Microfluidics, Inc., USA, of a laboratory or industrial type, there is at first microfluidized the powder lecithin of a pharmacological purity at concentration between 10 to 30 mg per ml of sterile isotonic solution. Microfluidization is conducted e.g. in the Z-chamber of 100 micrometer in diameter by several cycles so that the whole volume of fluid is by several times cycled across the microfluidization chamber, at a pressure of more than 1000 Bar. Afterwards, the curing powder or the treated curing powder FCH is added in the ratio of 2 to 1 related to the lecithin. Then the suspension is microfluidized on a microfluidizer in the Y-chamber of 100 or 75 micrometer in diameter by at least 100 cycle passages of the whole volume of fluid and a pressure of more than 1500 Bar and slush cooling. Then follows microfluidization on a microfluidizer in the Z-chamber of 100 micrometer in diameter by at least 100 cycle passages of the fluid at a pressure of more than 1500 Bar and slush cooling. During the last step, the suspension is again microfluidized on a microfluidizer in the Z-chamber of 50 micrometer in diameter by at least 100 cycle passages of the fluid at a pressure of more than 1500 Bar and slush cooling.

### Example #4

Lecithin or other lipid in the pharmacological purity at the concentration of 10 to 30 mg per milliliter of sterile isotonic solution is after solubilization treated by extrusion across the filters with sizes 100 to 500 nm. The resulting liposomes are then mixed with the curing drug or the treated curing drug according to the points 2 to 5 in the ratio of 5 : 1 to 0, 1 : 1 related to the lecithin (lipid) and is again treated by extrusion across the filters with sizes 100 to 500 nm. In the end, the resulting suspension may be treated as described in the above Examples #1 to #3.

### Examples of treatment of amorphous or powder curing drug:

### Example #5

Fine powder FCH is coated by glucose in a ratio of 5 - 10 % per one gram of FCH.

### Example #6

Fine powder FCH is coated by polyethyleneglycol PEG600 in a ratio of 5 - 10 % per one gram of FCH.

### Example #7

Fine powder FCH is coated by lecithin of a pharmacological purity in a ratio of 5 - 10 % per one gram of FCH.

### Example #8

Fine powder FCH is coated by sodium chloride (NaCl) in a ratio of 5 - 10 % per one gram of FCH.

### Example #9

Fine powder FCH is hydrated onto an aqueous paste containing 25% of dry FCH.

### Examples of mixing the gel with resulting liposomes:

### Example #10

4 % carboxymethylcellulose in sterile water
Preservative: 4 % Parabenum
Sterile water added up to 100 ml

### Example #11

4 % carboxymethylcellulose in sterile water
Preservative: 2 % Parabenum
Sterile water added up to 100 ml

### Industrial applicability

Liposomal gel hydrophobic phthalocyanine preparation is usable in medicine according this invention for therapy of tumors and other diseases.

## Claims

1. A method of preparation of a liposomal hydrophobic phthalocyanine gel preparation comprising the steps of:
fluidizing lecithin of pharmaceutical purity in a concentration from 1 to 40 mg per ml of sterile isotonic solution, wherein the whole volume of the fluid passes through a fluidizing chamber in a microfluidizer, to a final particle size smaller than 1000 nanometer at a temperature higher than 0 °C and at a pressure of 500 to 2000 bar;
adding, with constant stirring, an active substance in a ratio of 0.1 to 5:1 in relation to lecithin, the active substance being selected from the group consisting of hydrophobic hydroxyaluminum phthalocyanine, hydrophobic aluminum phthalocyanine, hydrophobic zinc phthalocyanine, hydrophobic silicone phthalocyanine, organic silicone phthalocyanine, and hydrophobic phthalocyanine without the core metal;
fluidizing the suspension in the microfluidizer to a final particle size smaller than 500 nanometer at a pressure of 1000 to 2000 bar and at a temperature higher than 0 °C; and
mixing the resulting suspension with a translucent gel in a ratio between 10:1 to 1:10.

2. The method of preparation of a liposomal hydrophobic phthalocyanine gel preparation according to claim 1, wherein the active substance is fluidized at least 100 cycles in the same amount of isotonic solution in relation to the lecithin, to a final particle size smaller than 1000 nanometer at a pressure in the range of 1000 to 2000 bar.

3. The method of preparation of a liposomal hydrophobic phthalocyanine gel preparation according to claim 1 or 2, wherein the active substance is coated with glucose in a ratio of 5 - 10 % per gram of active substance.

4. The method of preparation of a liposomal hydrophobic phthalocyanine gel preparation according to claim 1 or 2, wherein the active substance is coated with polyethylenglycol in a ratio of 5 - 10 % per gram of active substance.

5. The method of preparation of a liposomal hydrophobic phthalocyanine gel preparation according to claim 1 or 2, wherein the active substance is coated with lecithin in a ratio of 5 - 10 % per gram of active substance.

6. The method of preparation of a liposomal hydrophobic phthalocyanine gel preparation according to claim 1 or 2, wherein the active substance is coated with NaCl in a ratio of 5 - 10 % per gram of active substance.

## Patentansprüche

1. Verfahren zur Herstellung einer liposomalen hydrophoben Phthalocyanin-Gel-Zubereitung, umfassend die folgenden Schritte:
Fluidisieren von Lecithin von pharmazeutischer Reinheit in einer Konzentration von 1 bis 40 mg pro ml steriler isotoner Lösung, wobei das gesamte Volumen des Fluids durch eine Fluidisierungskammer in einem Mikrofluidisierer läuft, zu einer endgültigen Partikelgröße von weniger als 1.000 Nanometer bei einer Temperatur von mehr als 0 °C und bei einem Druck von 500 bis 2.000 bar;
Zusetzen, unter ständigem Rühren, eines Wirkstoffs in einem Verhältnis von 0,1 bis 5 zu 1 in Bezug auf Lecithin, wobei der Wirkstoff aus der Gruppe ausgewählt wird, die aus hydrophobem Hydroxyaluminium-Phthalocyanin, hydrophobem Aluminium-Phthalocyanin, hydrophobem Zink-Phthalocyanin, hydrophobem Silicium-Phthalocyanin, organischem Silicium-Phthalocyanin und hydrophobem Phthalocyanin ohne das Kernmetall besteht;
Fluidisieren der Suspension in dem Mikrofluidisierer zu einer endgültigen Partikelgröße von weniger als 500 Nanometer bei einem Druck von 1.000 bis 2.000 bar und bei einer Temperatur von mehr als 0 °C; und
Mischen der resultierenden Suspension mit einem lichtdurchlässigen Gel in einem Verhältnis zwischen 10:1 und 1:10.

2. Verfahren zur Herstellung einer liposomalen hydrophoben Phthalocyanin-Gel-Zubereitung nach Anspruch 1, wobei der Wirkstoff mit wenigstens 100 Zyklen in der gleichen Menge an isotoner Lösung in Bezug auf das Lecithin zu einer endgültigen Partikelgröße von weniger als 1.000 Nanometer bei einem Druck im Bereich von 1.000 bis 2.000 bar fluidisiert wird.

3. Verfahren zur Herstellung einer liposomalen hydrophoben Phthalocyanin-Gel-Zubereitung nach Anspruch 1 oder 2, wobei der Wirkstoff mit Glucose in einem Verhältnis von 5 bis 10 % pro Gramm Wirkstoff beschichtet wird.

4. Verfahren zur Herstellung einer liposomalen hydrophoben Phthalocyanin-Gel-Zubereitung nach Anspruch 1 oder 2, wobei der Wirkstoff mit Polyethylenglycol in einem Verhältnis von 5 bis 10 % pro Gramm Wirkstoff beschichtet wird.

5. Verfahren zur Herstellung einer liposomalen hydrophoben Phthalocyanin-Gel-Zubereitung nach Anspruch 1 oder 2, wobei der Wirkstoff mit Lecithin in einem Verhältnis von 5 bis 10 % pro Gramm Wirkstoff beschichtet wird.

6. Verfahren zur Herstellung einer liposomalen hydrophoben Phthalocyanin-Gel-Zubereitung nach Anspruch 1 oder 2, wobei der Wirkstoff mit NaCl in einem Verhältnis von 5 bis 10 % pro Gramm Wirkstoff beschichtet wird.

## Revendications

1. Procédé de préparation d'une préparation de gel de phtalocyanine hydrophobe liposomique comprenant les étapes de :
fluidification de lécithine de pureté pharmaceutique à une concentration de 1 à 40 mg par ml dans une solution isotonique stérile, où le volume entier du fluide passe à travers une chambre de fluidification dans un microfluidiseur, à une taille de particule finale plus petite que 1000 nanomètres à une température supérieure à 0 °C et à une pression de 500 à 2000 bars ;
addition, avec agitation constante, d'une substance active dans un rapport de 0,1 à 5 : 1 en relation à la lécithine, la substance active étant choisie dans le groupe constitué par la phtalocyanine d'hydroxyaluminium hydrophobe, la phtalocyanine d'aluminium hydrophobe, la phtalocyanine de zinc hydrophobe, la phatalocyanine de silicone hydrophobe, la phtalocyanine de silicone organique et la phtalocyanine hydrophobe sans le métal de coeur ;
fluidification de la suspension dans le microfluidiseur à une taille de particule finale plus petite que 500 nanomètres à une pression de 1000 à 2000 bars et à une température supérieure à 0 °C ; et
mélange de la suspension résultante avec un gel translucide dans un rapport entre 10 : 1 à 1 : 10.

2. Procédé de préparation d'une préparation de gel de phtalocyanine hydrophobe liposomique selon la revendication 1, dans lequel la substance active est fluidifiée au moins 100 cycles dans la même quantité de solution isotonique en relation à la lécithine, à une taille de particule finale plus petite que 1000 nanomètres à une pression dans la plage de 1000 à 2000 bars.

3. Procédé de préparation d'une préparation de gel de phtalocyanine hydrophobe liposomique selon la revendication 1 ou 2, dans lequel la substance active est revêtue avec du glucose dans un rapport de 5 à 10 % par gramme de substance active.

4. Procédé de préparation d'une préparation de gel de phtalocyanine hydrophobe liposomique selon la revendication 1 ou 2, dans lequel la substance active est revêtue avec du polyéthylèneglycol dans un rapport de 5 à 10 % par gramme de substance active.

5. Procédé de préparation d'une préparation de gel de phtalocyanine hydrophobe liposomique selon la revendication 1 ou 2, dans lequel la substance active est revêtue avec de la lécithine dans un rapport de 5 à 10 % de substance active.

6. Procédé de préparation d'une préparation de gel de phtalocyanine hydrophobe liposomique selon la revendication 1 ou 2, dans lequel la substance active est revêtue avec du NaCl dans un rapport de 5 à 10 % par gramme de substance active.
